# EUROPEAN PATENT SPECIFICATION

(11) **EP 2 928 522 B1**
(45) Date of publication and mention of the grant of the patent: **03.06.2020**
(21) Application number: 13801599.5
(22) Date of filing: 09.12.2013
(51) Int. Cl.: A61M 5/142, A61M 5/168, B65B 3/32, F04B 9/08, G01F 11/06, F04B 3/00, F04B 13/00

(54) **MEDICAL PUMP AND METHOD OF OPERATING THE SAME**
MEDIZINISCHE PUMPE UND BETRIEBSVERFAHREN DAFÜR
POMPE MÉDICALE ET SON PROCÉDÉ DE FONCTIONNEMENT

(30) Priority: 10.12.2012 EP 12196373
(43) Date of publication of application: 14.10.2015
(73) Proprietor: Sanofi-Aventis Deutschland GmbH, 65929 Frankfurt am Main (DE)
(72) Inventor: NESSEL, Christian, 65926 Frankfurt am Main (DE)
(74) Representative: Johnson, Stephen William
(86) International application number: PCT/EP2013/075949
(87) International publication number: WO 2014/090745

(56) References cited:
- EP-A1- 2 357 454
- FR-A- 782 769
- FR-A1- 2 564 525
- GB-A- 1 345 867
- US-A- 5 513 779
- US-A1- 2004 256 422

## Description

The present invention relates to a medical pump, for conveying a liquid. Also disclosed is a method of operating the same.

For example, medical pumps are known from EP 0 040 592 A2 and EP 0 530 773 A1. Pumps in general are known from GB 1 345 867 A, FR 2 564 525 A1, US 5,513,779, and FR 782 769 A.

It is an object of the present disclosure to provide an improved medical pump and to provide a method of operating the medical pump.

This object is achieved by the subject-matter of the independent claims. Advantageous embodiments and refinements are subject-matter of the dependent claims.

One aspect of the present disclosure relates to a medical pump for conveying a liquid. The medical pump comprises a chamber. The chamber comprises an inlet for receiving the liquid and an outlet. The medical pump further comprises a first plunger element which is movably arranged in the chamber and a second plunger element which is movably arranged in the chamber. The first plunger element and the second plunger element are movable with respect to one another to define a chamber space formed between them. The medical pump is operable and configured such that, when the chamber space is fluidly connected to the inlet, liquid is moved into the chamber space through the inlet by a relative movement of the first and the second plunger element away from each other and, when liquid is arranged in the chamber space and the chamber space is fluidly connected to the outlet, the liquid is removed from the medical pump through the outlet by a relative movement of the first and the second plunger element towards each other.

In an embodiment, the medical pump is operable so as to move or introduce the liquid or a subquantity thereof through the inlet into the chamber via an underpressure in the chamber generated between the first and the second plunger element by a relative movement of the first and the second plunger element which results in the size of the chamber space between the first and the second plunger element being increased when the chamber space is fluidly connected to the inlet.

Accordingly, the liquid can advantageously be introduced or moved into and/or removed from the chamber only by means of the relative movement of the first and the second plunger element without any further components or mechanisms being necessary.

The chamber may constitute a pump chamber. The chamber may be a tube or comprise a tube-like shape.

In an embodiment, the chamber space is displaceable within the chamber by a movement of the first and the second plunger element.

In an embodiment, the first and the second plunger element are arranged along a common longitudinal axis and the chamber space is axially displaceable by a simultaneous movement of the first and the second plunger element with respect to the chamber. The inlet and the outlet are arranged axially spaced apart from each other. The first and the second plunger element may be arranged oppositely. According to this embodiment, it is possible to displace the chamber space within the chamber such that the chamber space may be fluidly connected to or fluidly disconnected from the inlet and the outlet. During the simultaneous movement of the first and the second plunger element, the size or volume of the chamber space is preferably kept constant.

In an embodiment, the chamber space is fluidly connectable to and fluidly disconnectable from the inlet and the outlet by movement of at least one of or both of the first and the second plunger element with respect to the chamber. This provides the advantage that the chamber space and/or the chamber may selectively either be sealed within the medical pump or communicate with, e.g., an exterior of the medical pump via the inlet and the outlet. Preferably, the chamber space and/or the chamber are liquid and gas tight against the exterior of the medical pump, when the chamber space and/or the chamber are sealed against the exterior of the medical pump. In this respect, the first and the second plunger element are preferably sealingly connected to or retained with respect to the chamber.

In an embodiment, the chamber comprises an opening which is arranged axially spaced from the inlet and the outlet, wherein the chamber space is fluidly connectable to and fluidly disconnectable from the opening by movement of at least one of or both of the first and the second plunger element with respect to the chamber. This provides the advantage that the chamber space and/or the chamber may selectively either be sealed within the medical pump or communicate with, e.g., an exterior of the medical pump via the opening.

In an embodiment, the medical pump comprises a substance reservoir which may be fluidly connectable to the chamber space, the substance reservoir containing a liquid with a medical substance.

In an embodiment, the medical pump comprises a detection mechanism operable to detect whether a content of the chamber space which content comprises the liquid fulfils a predetermined compression property requirement. By way of the detection mechanism, it may be detected, if a critical amount of gas, as e.g. gas bubbles, is present in the chamber space along with the liquid. In this regard, a content of the chamber space may comprise liquid with the medical substance and possibly also gas. Such gas may pose a significant health risk to a patient, particularly, when the medical substance is being dispensed to the patient along with the gas. The gas may have entered the chamber space, e.g. when a subquantity of the liquid is moved into the chamber space from the substance reservoir. By means of the detection mechanism, it can be assessed whether a critical amount of gas is present in the chamber space.

In this respect, the predetermined compression property requirement may relate to the compressibility of the content of the chamber space including the subquantity of the liquid and possibly gas. The content of the chamber space comprises a greater compressibility if it comprises gas, as compared to the situation, wherein no or substantially no gas is present in the chamber space along with the liquid. User safety of the medical pump may be increased in that, depending on the fulfilment of the predetermined compression property requirement, the subquantity of the liquid (possibly comprising gas) can be dispensed or not by the medical pump.

Operation of the detection mechanism may utilize the first and/or the second plunger element, in particular movement of the first and/or the second plunger element.

In an embodiment, the medical pump comprises a housing which defines or houses the chamber.

The inlet is preferably arranged on a side of the chamber which is opposite to the side at which the outlet and the opening are arranged. Preferably, the inlet is arranged at an axial position between the opening and the outlet. According to this embodiment, a compact design of the medical pump can be achieved.

According to this embodiment, the first plunger element is preferably provisioned to fluidly connect and/or fluidly disconnect the chamber space from the outlet and/or the inlet and the second plunger element is preferably provisioned to fluidly connect and/or disconnect the chamber space from the opening and/or the inlet.

In an embodiment, the medical pump is an infusion pump.

In an embodiment, the outlet is a dispensing outlet.

The medical pump can be operated to convey a medical substance from the substance reservoir into the chamber space and then through the dispensing outlet. Moreover, the medical pump may be operated such that, e.g., a subquantity of the liquid is conveyed or dispensed continuously, i.e. one subquantity is conveyed and dispensed after the other, while the user is continuously connected to the medical pump, e.g. via a needle. The liquid and/or the medical substance may be dispensed through the dispensing outlet via a relative movement of the first and the second plunger element towards each other.

In an embodiment, the medical pump comprises a needle or a needle assembly which may be fluidly connected with the chamber via the dispensing outlet, e.g. via a tube. According to this embodiment, the liquid with the medical substance can advantageously be dispensed through the needle or the needle assembly.

It is an advantage of the medical pump that it consists of a comparably low number of interacting parts. In this way the medical pump is embodied robust and cost efficient.

In an embodiment, the medical pump is configured such that the detection mechanism is operated, when the chamber space is fluidly disconnected from the outlet. This provides the advantage that upon a compression which may occur during the operation of the detection mechanism, liquid is prevented from being removed from the chamber through the outlet. Preferably, the chamber space is fluidly disconnected or sealed from an exterior of the chamber, when the detection mechanism is operated.

In an embodiment, the content of the chamber space is compressed by a relative movement of the first and the second plunger element with respect to each other, when the detection mechanism is operated. This relative movement tends to reduce or reduces the size of the chamber space. Thereby, the content of the chamber space may be compressed. This embodiment provides the advantage that a pressure may be applied to the content of the chamber space by the first and the second plunger element. Thereby it may be assessed or detected whether the content of the chamber space fulfils the predetermined compression property requirement. For example, it may be assessed in this way whether the content of the chamber space can be compressed to a certain extent, when a given pressure or force is applied to the content of the chamber space.

In an embodiment, the predetermined compression property requirement is fulfilled, when, during the operation of the detection mechanism, the content of the chamber space is compressed only up to a predetermined extent.

In an embodiment, the predetermined compression property requirement is not fulfilled, when, during the operation of the detection mechanism, the content of the chamber space is compressible more than the predetermined extent.

In an embodiment, the predetermined extent is chosen such that, when the content of the chamber space is compressible more than the predetermined extent, a critical amount of gas is expected to be present in the chamber space along with the subquantity of the liquid. As an advantage thereof, a threshold can be defined by the predetermined extent which distinguishes whether a critical or hazardous amount of gas is present in the chamber space.

In an embodiment, at least one of or both of the first and second plunger element is selectively lockable with respect to the chamber such that when the respective plunger element is locked, relative movement of that plunger element with respect to the chamber is prevented. This provides the advantage that only one of the first and the second plunger element has to be moved with respect to the other one, e.g. during the operation of the detection mechanism in order to compress the content of the chamber space.

Preferably, every time when one of the first and the second plunger element is moved relatively with respect to the other one, only one of the first and the second plunger element is moved and the other one is locked.

In an embodiment, the medical pump is operable so as to dispense the subquantity through the outlet if the predetermined compression property requirement is fulfilled. Thereby, it may be assured that the liquid is only dispensed from the medical pump when there is no or substantially no gas present in the chamber space along with the liquid.

In an embodiment, the liquid is dispensed by relative movement of the first and the second plunger element which reduces the size of the chamber space when the substance space is fluidly connected to the outlet. In this embodiment the liquid can advantageously be dispensed only by means of the relative movement of the first and the second plunger element without any further components or mechanisms being necessary.

In an embodiment, the medical pump is configured such that the detection mechanism is operated when the chamber space is fluidly disconnected from the opening. This provides the advantage that upon a compression of the content of the chamber space, during the operation of the detection mechanism, liquid is prevented from being removed from the chamber through the opening. Preferably, the chamber space is fluidly disconnected or sealed from an exterior of the chamber, when the detection mechanism is operated.

In an embodiment, the content of the chamber space is removed by a relative movement of the first and the second plunger element which reduces the size of the chamber space when the chamber space is fluidly connected to the opening. In this embodiment the chamber space can advantageously be emptied only by means of the relative movement of the first and the second plunger element without any further components or mechanisms being necessary.

In an embodiment, the medical pump is configured such that the detection mechanism is operated when the chamber space is fluidly disconnected from the inlet. This provides the advantage that upon a compression during the operation of the detection mechanism, liquid is prevented from being removed from the chamber through the inlet. Preferably, the chamber space is fluidly disconnected or sealed from an exterior of the chamber when the detection mechanism is operated.

In an embodiment, the medical pump is operable so as to introduce the subquantity from the substance reservoir through the inlet into the chamber via an underpressure in the chamber generated between the first and the second plunger element by a relative movement of the first and the second plunger element which results in the size of the chamber space between the first and the second plunger element being increased, when the chamber space is fluidly connected to the inlet. In this embodiment the liquid can advantageously be introduced only by means of the relative movement of the first and the second plunger element without any further components or mechanisms being necessary.

In an embodiment, the first and the second plunger element are movable along the longitudinal axis. Thereby, the medical pump may be embodied simple and robust. This is due to a comparably low number of interacting components involved in the function of the medical pump. Moreover, a sealing of the chamber or, as the case may be, the chamber space against an exterior of the chamber, may be eased in this way.

In an embodiment, the outlet and the opening are arranged axially spaced apart from each other, wherein the inlet is arranged at an axial position between the outlet and the opening. According to this embodiment, a compact design of the medical pump can be achieved.

In an embodiment, the outlet and the opening are arranged at a first side of the chamber and the inlet is arranged at a second side of the chamber which opposes the first side. According to this embodiment, further components of the medical pump which are arranged which may be arranged at the second side, while, for example, the needle or any additional components which may be provisioned when the content of the chamber space is dispensed or removed from the chamber or from the chamber space can expediently be arranged at the first side in order not to interact with the further components.

In an embodiment, the medical pump comprises two motors which are operable to independently control the movement of the first and the second plunger element within the chamber. As an advantage, the first and the second plunger element may be independently moved by the motors, particularly along the laxis. Thereby, the medical pump may be configured such that a plunger element is only moved when the respective motor driving this plunger element is activated, while, when the motor is not activated, the respective plunger element is locked. Additionally or alternatively, a separate locking mechanism operable to lock the first and the second plunger element may be provided.

Preferably, the compression of the content of the chamber space via the first and the second plunger element during the operation of the detection mechanism is also carried out via the motors. In this respect a control unit may be provisioned which may control the movement of the motors, when the content of the chamber space is being compressed.

In an embodiment, the medical pump is configured such that the detection mechanism is operated when the chamber space is fluidly disconnected from the exterior of the chamber. This provides the advantage that upon a compression of the content of the chamber space, during the operation of the detection mechanism, liquid is prevented from being removed from the chamber. Preferably, the chamber space is fluidly disconnected or sealed from an exterior of the chamber, when the detection mechanism is operated.

In an embodiment, the medical pump is operable so as to empty the chamber space through the opening if the predetermined compression property requirement is not fulfilled, and wherein the content of the chamber space is removed by a relative movement of the first and the second plunger element. As an advantage, the content of the chamber space can be removed from the chamber in the case that the predetermined compression property requirement is not fulfilled such that afterwards a new subquantity of the liquid may be introduced from the substance reservoir into the chamber space.

In an embodiment, the inlet is arranged at an axial position between the outlet and the opening, and wherein the detection mechanism is operated when the chamber space is axially arranged between the inlet and the outlet. When the predetermined compression property requirement is fulfilled, the liquid may then directly be dispensed through the outlet without being exposed or fluidly connected to the inlet or the opening again. This provides the advantage that the subquantity or dose of the liquid is not falsified before being dispensed, as no liquid may escape through the inlet or the opening.

In an embodiment, the opening is a further inlet and the medical pump is operable and configured such that a further liquid is moved into the chamber space through the opening by a relative movement of the first and the second plunger element away from each other when the chamber space is fluidly connected to the opening. According to this embodiment, the substance reservoir may comprise the further liquid and the substance reservoir may be arranged such that it is fluidly connectable to the opening. The opening is preferably arranged at a side, e.g. a longitudinal side of the chamber at which also the inlet is arranged.

The outlet is preferably arranged on a side of the chamber which is opposite to the side at which the inlet and the opening are arranged. Preferably, the outlet is arranged at an axial position between the inlet and the opening. According to this embodiment, a compact design of the medical pump can be achieved.

According to this embodiment, the first plunger element is preferably provisioned to fluidly connect and/or disconnect the chamber space from the inlet and/or the outlet and the second plunger element is preferably provisioned to fluidly connect and/or disconnect the chamber space from the opening and/or the outlet.

In an embodiment, the medical pump is operable so as to move or introduce the further liquid or a subquantity thereof from the substance reservoir through the further inlet into the chamber via an underpressure in the chamber generated between the first and the second plunger element by a relative movement of the first and the second plunger element which results in the size of the chamber space between the first and the second plunger element being increased, when the chamber space is fluidly connected to the further inlet. In this embodiment, the further liquid can advantageously be introduced only by means of the relative movement of the first and the second plunger element without any further components or mechanisms being necessary. Two different liquids may thus be subsequently moved into the chamber space from two different inlets, whereby the medical pump additionally allows the two different liquids to mix and/or react, such as chemically react in the chamber space.

In an embodiment, the medical pump comprises a sensor unit which is fluidly connected to the outlet. The medical pump is operable and configured such that when the liquid and the further liquid have been moved into the chamber space, the liquid and the further liquid mix and wherein the corresponding mixture of the liquid and the further liquid is moved into the sensor unit.

In an embodiment, the liquid may be a body fluid.

In an embodiment, the further liquid may be a reactant or reagent which may be suitable such that a blood glucose measurement may be performed by the sensor unit. The sensor unit may be configured accordingly.
A further aspect of the present disclosure relates to a method of operating a medical pump. The method comprises the steps of moving a liquid into the chamber of the medical pump through the inlet of the chamber by a relative movement of the first and the second plunger element of the medical pump away from each other such that the liquid is retained in the chamber space. The chamber space is formed between the first and the second plunger element. The method comprises displacing the chamber space within the chamber by a simultaneous movement of the first and the second plunger element with respect to the chamber. The method further comprises removing the liquid from the medical pump by a relative movement of the first and the second plunger element towards each other.

In an embodiment of the method, the displacement of the chamber space within the chamber is performed when the chamber space is fluidly disconnected from the exterior of the chamber. The method further comprises detecting of whether the content of the chamber space fulfils the predetermined compression property requirement.

In an embodiment, the method comprises fluidly connecting the chamber space to the outlet of the chamber by moving the chamber space to the outlet of the chamber and dispensing the content of the chamber space through the outlet, if the predetermined compression property requirement is fulfilled.

In an embodiment, the method comprises fluidly connecting the chamber space to the opening of the chamber by moving the chamber space to the opening of the chamber and emptying the chamber space through the opening, if the predetermined compression property requirement is not fulfilled.

The detection of whether the content of the chamber space fulfils the predetermined compression property requirement is preferably carried out when the chamber space is fluidly disconnected or sealed from the exterior of the chamber.

In an embodiment of the method, the method comprises moving a liquid into a chamber of the medical pump through an inlet of the chamber such that the liquid is retained in a chamber space formed between the first and the second plunger element. The method further comprises displacing the chamber space within the chamber such that the chamber space is fluidly disconnected from the inlet and fluidly connected to the further inlet of the chamber. The method further comprises moving the further liquid into the chamber through the further inlet of the chamber such that further liquid is retained in the chamber space. The method further comprises displacing the substance space within the chamber such that the chamber space is fluidly disconnected from the inlet and the further inlet and fluidly connected to the outlet of the chamber. The method further comprises emptying the chamber space through the outlet by a relative movement of the first and the second plunger element towards each other. The features and components which are described herein above and below in conjunction with different aspects or embodiments may also apply for other aspects and embodiments. Particularly, features and components described with respect to the medical pump may apply for the method of operation and vice versa.

In an embodiment, the medical pump comprises a chamber further comprising an inlet for receiving the liquid and an outlet. The medical pump further comprises a first plunger element movably arranged in the chamber and a second plunger element movably arranged in the chamber, the first plunger element and the second plunger element being movable with respect to one another to define a chamber space formed between them, wherein the chamber space is displaceable within the chamber by a movement of the first and the second plunger element, wherein the first and the second plunger element are arranged along a common longitudinal axis and the chamber space is axially displaceable by a simultaneous movement of the first and the second plunger element with respect to the chamber, wherein the inlet and the outlet are arranged axially spaced apart from each other. The chamber comprises an opening which is arranged axially spaced from the inlet and the outlet, wherein the chamber space is fluidly connectable to and fluidly disconnectable from the opening by movement of at least one of or both of the first and the second plunger element with respect to the chamber. The medical pump is further operable and configured such that, when the chamber space is fluidly connected to the inlet, liquid is moved into the chamber space through the inlet by a relative movement of the first and the second plunger element away from each other, and, when liquid is arranged in the chamber space and the chamber space is fluidly connected to the outlet, the liquid is removed from the medical pump through the outlet by a relative movement of the first and the second plunger element towards each other.

Further features and advantages of the subject-matter of this disclosure will become apparent from the following description of the exemplary embodiment in conjunction with the figures, in which:
Figure 1 shows a schematic diagram of a medical pump by means of a cross-sectional view according to a first embodiment;
Figure 2 illustrates a method of operating the medical pump shown in Figure 1, when the predetermined compression property requirement is fulfilled;
Figure 3 describes the method of operating the medical pump shown in Figure 1, when the predetermined compression property requirement is not fulfilled;
Figure 4 shows a schematic diagram of a medical pump by means of a cross-sectional view according to a second embodiment;
Figure 5 describes a further method of operating the medical pump shown in Figure 4.

Like elements, elements of the same kind and identically acting elements may be provided with the same reference numerals in the figures. Additionally, the figures may be not true to scale. Rather, certain features may be depicted in an exaggerated fashion for better illustration of important principles.

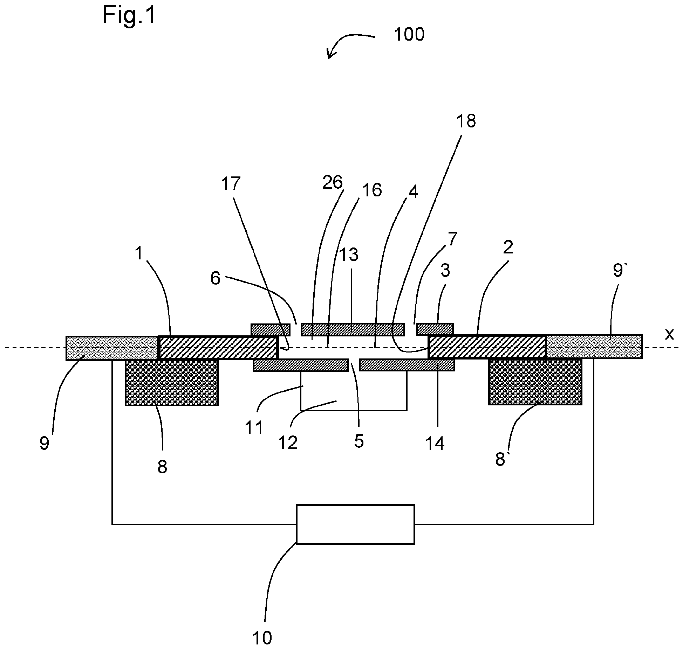

Figure 1 shows in a first embodiment a medical pump 100 comprising a first plunger element 1, a second plunger element 2 and a housing 3. The medical pump 100 may be operated as an infusion pump for delivering, particularly continuously delivering a subquantity 15 of a liquid 12 comprising a medical substance. The housing 3 defines a chamber 4 in which the first and the second plunger element 1, 2 are oppositely arranged, thereby being movable along a longitudinal axis x. The chamber 4 comprises an inlet 5, a dispensing outlet 6 and a non-dispensing outlet 7. The non-dispensing outlet 7 may be an opening of the chamber 4. Furthermore, the medical pump 100 comprises two stators 8 and 8' and two motors 9 and 9'. The stator 8 and the motor 9 are arranged on the side of the medical pump 100 on which the first plunger element 1 is arranged. The stator 8' and the motor 9' are arranged on the side of the medical pump 100 on which the second plunger element 2 is arranged. The motor 9 is preferably configured to drive the first plunger element 1 with respect to the housing 3 or the chamber 4 and the motor 9' is preferably configured to drive the second plunger element 2 with respect to the housing 3 or the chamber 4.

The medical pump 100 further comprises a control unit 10 which controls the motors 9 and 9'. Moreover, the medical pump 100 is configured such that the first and the second plunger element 1, 2 can selectively be locked with respect to the housing 3 or the chamber 4.

The medical pump 100 comprises a substance reservoir 11 containing a liquid 12 with a medical. The dispensing outlet 6 and the non-dispensing outlet 7 are arranged at a first side 13 of the chamber 4 and the inlet 5 is arranged at a second side 14 of the chamber 4 which is opposite from the dispensing outlet 6 and the non-dispensing outlet 7. Moreover, the inlet 5 is arranged at an axial position between the dispensing outlet 6 and the non-dispensing outlet 7.

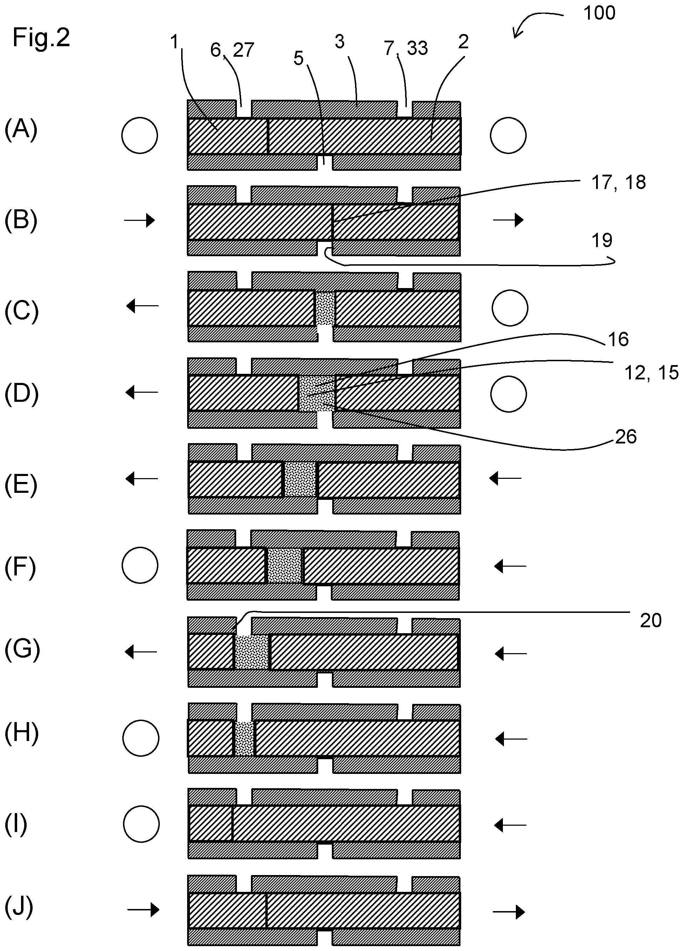

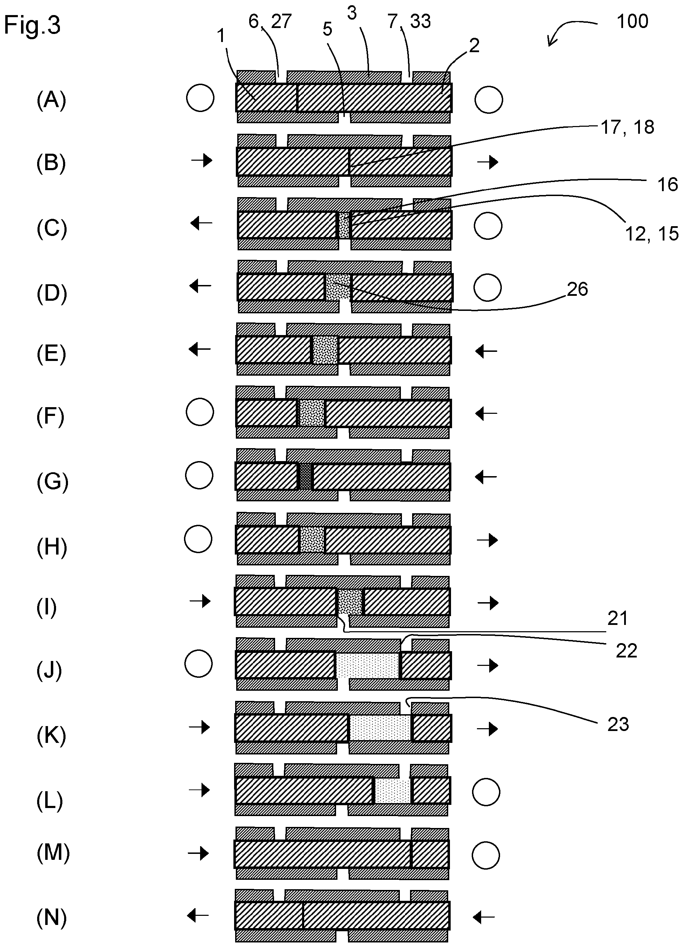

The medical pump 100 is operable to move a subquantity 15 (cf. also Figures 2 and 3) of the liquid 12 from the substance reservoir 11 into a chamber space 16 through the inlet 5 which may be fluidly connected to the substance reservoir 11. The chamber space 16 may be formed within the chamber 4 between the first plunger element 1 and the second plunger element 2.

For the sake of clarity only, not all of the components of the medical pump 100 are shown in the Figures 2 and 3.

By means of Figure 2 a method of operating the medical pump 100 as an infusion pump is described. Particularly, it is shown in Figure 2 how the subquantity 15 of liquid 12 is introduced into the chamber space 16 and conveyed or dispensed through the dispensing outlet 6 after is it has been detected or assessed by a detection mechanism whether the content 26 of the chamber space 16 fulfils the predetermined compression property requirement (cf. Figure 2F). The medical pump 100 may comprise a tube and a needle or a needle unit (not explicitly indicated) through which the liquid 12 may be introduced. The needle or the needle unit may be connected to the dispensing outlet 6 via the tube on the first side 13 of the medical pump 100 such that said components are fluidly connected to the chamber 4 via the tube and the dispensing outlet 6. To this effect, the needle or needle unit may be configured such that the subquantity 15 is infused or dispensed to a patient through said needle or needle unit.

Figure 2A shows the medical pump 100, wherein the first and the second plunger element 1, 2 abut at an axial position between the inlet 5 and the dispensing outlet 6. The circles on each side of the medical pump 100 indicate that the plunger element which is arranged on the respective side is actually not moved or has not been moved in the preceding step. On the other hand, the arrows on each side of the medical pump 100 indicate the direction in which the plunger element, which is arranged on the respective side, is actually moved or has been moved in the preceding step.

Figure 2B also shows the medical pump 100. The arrows on both lateral sides of the medical pump 100 direct to the right thus indicating that the first and the second plunger element 1, 2 have been simultaneously moved to the right, i.e. towards the non-dispensing outlet 7, as compared to Figure 2A. The first and the second plunger element 1, 2 have been moved by the motors 9 and 9', respectively, such that an inner side face 17 of the first plunger element 1 and an inner side face 18 of the second plunger element 2 abut at an axial position which is aligned with a right side face 19 of the inlet 5. The right side face 19 of the inlet 5 is directed towards the non-dispensing outlet 7. The first and the second plunger element 1, 2 are driven by the motors 9 and 9', respectively. Moreover, the motors 9 and 9' are operable to independently control the movement of the first and the second plunger element 1, 2, respectively, within the chamber 4.

Figure 2C shows a situation of the medical pump 100 in which the first plunger element 1 has been moved to the left, i.e. away from the second plunger element 2, as compared to Figure 2B while the second plunger element 2 has not been moved, but has been locked. As a consequence, liquid 12 of the substance reservoir 11 is introduced into the chamber 4 through the inlet 5 via an underpressure which is generated in the chamber 4 between the first and the second plunger element 1, 2.

In Figure 2D the first plunger element 1 is further moved to the left while the second plunger element 2 is still locked.

The plunger element 1 is moved in Figure 2D until the chamber space 16 between the first and the second plunger element 1, 2 is filled with a subquantity 15 of the liquid 12. The subquantity 15 may constitute a defined volume which is to be continuously conveyed or dispensed or a dose of the liquid 12 or medical substance which is to be dispensed by the medical pump 100. The substance reservoir 11 may hold an amount of liquid 12 sufficient for dispensing a plurality of subquantities or doses. The subquantity 15 may further be adjusted by the control unit 10 via varying the distance the first plunger element 1 is moved with respect to the second plunger element 2 and, particularly with respect to the housing 3. The greater the distance is, the greater is or becomes the subquantity 15.

Subsequently, the chamber space 16 is displaced to the left in that the first and the second plunger element 1, 2 are moved to the left, i.e. towards the dispensing outlet 6, in a simultaneous manner (cf. Figure 2E). This movement is performed until the chamber space 16 is located at an axial position between the inlet 5 and the dispensing outlet 6 such that the chamber space 16 is fluidly disconnected from the inlet 5 and the dispensing outlet 6, as shown in Figure 2F. Of course, then, the chamber space 16 is also fluidly disconnected from the non-dispensing outlet 7. To this effect, the axial distance between the inlet 5 and the dispensing outlet 6 is expediently greater than the axial extension of the chamber space 16.

In this position of the chamber space 16, the detection mechanism is operated in order to detect whether the content 26 of the chamber space 16 fulfils the predetermined compression property requirement. Thereby, the content 26 of the chamber space 16 is compressed by a relative movement of the first and the second plunger element 1, 2 with respect to each other. Preferably, now, the first plunger element 1 is locked and the second plunger element 2 is moved left, i.e. towards the first plunger element 1. Alternatively, the second plunger element 2 may be locked and the first plunger element 1 may be moved right, i.e. towards the second plunger element 2. By means of the detection whether the content 26 of the chamber space 16 fulfils the predetermined compression property requirement, it may be assessed if gas or gas bubbles are present in the chamber space 16 along with the subquantity 15. Such gas may have entered the chamber 4 during the introduction of the subquantity 15, due to leakage or gas bubbles that were present in the substance reservoir.

The relative movement of the first and the second plunger element 1, 2 tends to reduce or reduces the size of the chamber space 16. The predetermined compression property requirement is fulfilled, when the content 26 of the chamber space 16 is compressed only up to a predetermined extent. The predetermined extent may relate to a defined volume of the chamber space 16 to which the content 26 of the chamber space 16 may just be compressed, when no or substantially no, expediently no hazardous amount of gas is present in the chamber space 16. As said volume depends on the force or the pressure by which the first and the second plunger element 1, 2 are moved relatively towards each other, the compression of the content 26 of the chamber space 16 may be carried out at a given force or pressure of the first and the second plunger element 1, 2.

Similarly, the predetermined extent may relate or be determined by the axial distance by which the first and the second plunger element 1, 2 are moved towards each other at a given force.

Alternatively, the predetermined extent may relate or be determined via the force necessary to move one of the first and the second plunger element 1, 2 a given axial distance towards the other one. Said given axial distance would then define a given size or volume of the chamber space 16.

If a critical or hazardous amount of gas is present in the chamber space 16, the content 26 of the chamber space 16 is compressed more than the predetermined extent, upon relative movement of the first and the second plunger element 1, 2. This is due to a greater compressibility of the content 26 of the chamber space 16 comprising gas, as gas has a compressibility which is considerably greater than the one of liquid. Depending on the speed of the movement of the first and the second plunger movement, the compressibility may be the isothermal compressibility or the adiabatic compressibility.

Said distance, volume or force may be monitored by the control unit 10 or an additional element or gauge of the medical pump during the operation of the detection mechanism. To this effect, the control unit 10 may compare the respective variable chosen from distance, volume or force, as mentioned above, to a threshold value which is stored by the control unit 10 and separates acceptable values of the respective variable from non-acceptable values. For example, if said distance or volume which is reached during the operation of the detection mechanism is equal to or greater than the threshold value, then, the predetermined compression property requirement is fulfilled, as the content 26 of the chamber space 16 is not compressed up to the predetermined extent. When, in this regard, said distance or volume which is reached during the operation of the detection mechanism is smaller than the predetermined extent, then, the predetermined compression property requirement is not fulfilled, as the content 26 of the chamber space 16 is compressed more than to the predetermined extent.

If the predetermined extent is determined by said force, then, the predetermined compression property requirement is fulfilled for forces equal to or greater than the threshold value. Accordingly, the predetermined compression property requirement is not fulfilled for forces smaller than the threshold value.

In the present case, in Figure 2F, the subquantity 15 within the chamber space 16 is sufficiently free of gas, so that the predetermined compression property requirement is fulfilled, as the content 26 of the chamber space 16 is or cannot be compressed significantly by the first and the second plunger element 1, 2. This is due to the limited compressibility of the content 26 of the chamber space 16 substantially containing the subquantity 15 of the liquid 12. Hence, the subquantity 15 may be dispensed by the medical pump 100.

In Figure 2G the chamber space 16 is moved to the left, i.e. towards the dispensing outlet 6 by a simultaneous movement of the first and the second plunger element 1, 2 such that the chamber space 16 is fluidly connected to the dispensing outlet 6 in order to dispense the subquantity 15. The simultaneous movement is carried out until the inner side face 17 of the first plunger element 1 is aligned to a left side face 20 of the dispensing outlet 6. The left side face 20 of the dispensing outlet 6 is directed away from the inlet 5 and the non-dispensing outlet 7. Then, preferably the first plunger element 1 is locked while the second plunger element 2 is moved further to the left in order to urge the subquantity 15 out of the chamber 4 through the dispensing outlet 6 (cf. Figure 2H). Thereby, the subquantity 16 is dispensed from the medical pump 100.

Figure 2I shows that the second plunger element 2 has been still further moved to the left such that the subquantity 15 has been completely urged from the chamber 4 and the inner side face 18 of the second plunger element 2 abuts the inner side face 17 of the first plunger element 1.

In Figure 2J, the first and the second plunger element 1, 2 are moved simultaneously to the right and into the initial position, again. Said movement to the right, i.e. towards the non-dispensing outlet 7 may be carried out to prepare the medical pump 100 to introduce a subsequent subquantity from the substance reservoir to the chamber 4.

Figure 3 illustrates a method of operation of the medical pump 100, wherein the steps depicted in the Figures 3A to 3F are similar to the Figures 2A to 2F. As compared to the Figure 2F, the content 26 of the chamber space 16 can be compressed in Figure 3F more than the predetermined extent, upon relative movement of the first and the second plunger element 1, 2 towards each other. As a consequence, the detection mechanism detects that the content 26 of the chamber space 16 does not fulfil the predetermined compression property requirement, as gas or air is present in the chamber space 16. Figure 3G illustrates that the size or volume of the chamber space 16 is significantly decreased, as compared to the Figure 3F. Consequently, the subquantity 15 is not dispensed by the medical pump 100, as the gas in the chamber space 16 would also be dispensed then.

Instead, when the second plunger element 2 has been moved to the left, i.e. towards the first plunger element 1 in order to reach its original position it had before the compression (cf. Figure 3H), the content 26 of the chamber space 16 is not being compressed anymore. Then, the chamber space 16 is moved to the right, i.e. towards the non-dispensing outlet 7 by a simultaneous movement of the first and the second plunger element 1, 2 until the inner side face 17 of the first plunger element 1 is axially aligned with a left side face 21 of the inlet 5 (cf. Figure 3I). The left side face 21 of the inlet 5 is directed towards the dispensing outlet 6. Now, the chamber space 16 is fluidly connected to the inlet 5.

Then, the first plunger element 1 is locked at the mentioned position and the second plunger element 2 is further moved to the right and away from the first plunger element 1. As the chamber space 16 is fluidly connected to the inlet 5, liquid 12, potentially along with further residual gas or air inside the substance reservoir, is moved into the chamber space 16 by an underpressure in the chamber 4 generated between the first and the second plunger element 1, 2. The second plunger element 2 is moved until the inner side face 18 of the second plunger element 2 is aligned with a left side face 22 of the non-dispensing outlet 7 (cf. Figure 3J). The left side face 22 of the non-dispensing outlet 7 is directed towards the inlet 5 and the dispensing outlet 6.

At this time, a maximum volume of liquid-gas mixture or fluid is held in the chamber space, wherein the chamber space is still fluidly disconnected from the non-dispensing outlet 7. In Figure 3K, the first and the second plunger element 1, 2 are moved simultaneously to the right, i.e. towards the non-dispensing outlet 7, until the inner side face 17 of the first plunger element 1 is aligned with a right side face 19 of the inlet and until the inner side face 18 of the second plunger element 2 is aligned with a right side face 23 of the non-dispensing outlet 7. The right side face 23 of the non-dispensing outlet 7 is directed away from the inlet 5 and the dispensing outlet 6. Then, as shown in Figure 3L, the second plunger element 2 is locked and the first plunger element 1 is moved right, i.e. towards the second plunger element 2, in order to empty the chamber space 16. In Figure 3M, the inner side face 17 of the first plunger element 1 abuts the inner side face 18 of the second plunger element 2 and the chamber space 16 is completely emptied. Now, the medical pump 100 may be prepared to be operated again. It is an advantage of the mentioned functionality that gas present in the substance reservoir may be removed from the substance reservoir in the vicinity of the inlet 5. In Figure 3N, the first and the second plunger element 1, 2 are simultaneously moved to the left, as compared to the Figure 3M.

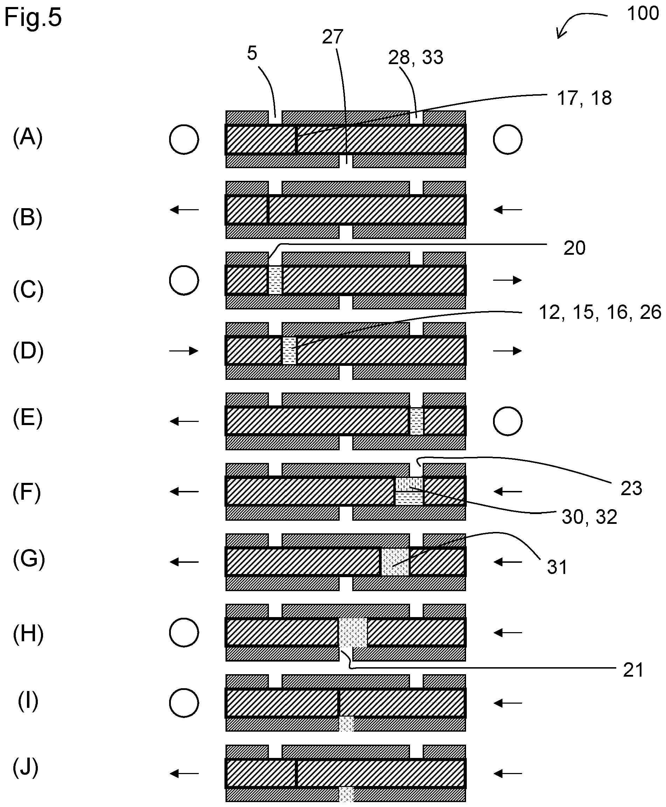

Figure 5 shows a second embodiment of a medical pump 100 comparable to the one shown in Figure 1. The medical pump 100 may be operated as a pump for conveying liquids. The chamber 4 comprises an inlet 5, a further inlet 28 and an outlet 27. In contrast to the medical pump shown in Figure 1, the substance reservoir containing a further liquid 30 is fluidly connected to the further inlet 28. Moreover, the inlet 5 and the further inlet 28 are arranged at the first side 13 of the chamber 4 and the outlet 27 is arranged at the second side 14 of the chamber. The outlet 27 is arranged at an axial position between the inlet 5 and the further inlet 28. The medical pump 100 further comprises a sensor unit 29 which is fluidly connected to the outlet 27.

For the sake of clarity only, not all of the components of the medical pump 100 are shown in the Figure 5.

By means of Figure 5 a method of operating the medical pump 100 is described, wherein the medical pump 100 is operated or used for conveying one or more liquids. Particularly, it is shown in Figure 5 that a subquantity 15 of a liquid 12 is introduced into the chamber space 16 through the inlet 5. The subquantity 15 may differ from the above mentioned suquantity, e.g. in terms of volume. In this embodiment, the liquid 12 is preferably a body fluid. Said body fluid, as e.g. blood may be introduced into the chamber space 16 via a needle and/or or a tube which may be fluidly connected to the inlet 5. Subsequently, a further subquantity 32 of a further liquid 30, preferably a reactant or reagent is introduced into the chamber space 16 through the further inlet 28. A mixture of the liquid 12 and the further liquid 30 may then be removed from the chamber space 16 through an outlet 27 and moved into the sensor unit, wherein the liquid 12 and the further liquid 30 react, as e.g. chemically react. Such a reaction may also already take place in the chamber 4. The liquid 12 and the further liquid 30 may be analysed in the sensor unit 29. The further liquid 30 may be a reagent or reactant and/or comprise a medical substance which may e.g. be suitable to react with the liquid 12 in order to allow for a blood glucose measurement. The sensor unit 29 may be suitable to perform such a blood glucose measurement.

Figure 5A shows the medical pump 100, wherein the first and the second plunger element 1, 2 abut at an axial position between the inlet 5 and an outlet 27. In Figure 5B, the first and the second plunger element 1, 2 have been simultaneously moved left, i.e. towards the inlet 5 by the motors 9 and 9', respectively, such that an inner side face 17 of the first plunger element 1 and an inner side face 18 of the second plunger element 2 abut at an axial position which is aligned with a left side face 20 of the inlet 5. Figure 5C shows a situation of the medical pump 100 in which the second plunger element 2 has been moved to the right, i.e. away from the first plunger element 1, as compared to Figure 5B while the first plunger element 1 has not been moved, but has been locked. As a consequence, liquid 12 is introduced or sucked into the chamber 4 through the inlet 5 via an underpressure which is generated in the chamber 4 between the first and the second plunger element 1, 2.

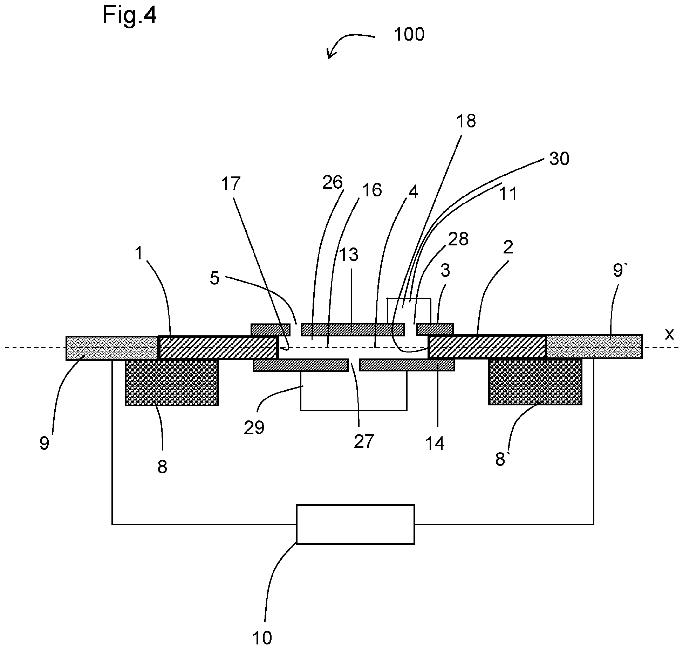

The second plunger element 2 is moved in Figure 5D until the chamber space 16 between the first and the second plunger element 1, 2 is filled with a subquantity 15 of the liquid 12. The subquantity 15 of liquid 12 may constitute a defined volume of body fluid, as e.g. blood. The subquantity 15 may be adjusted by the control unit 10 via varying the distance, the second plunger element 2 is moved with respect to the first plunger element 1. Subsequently, the chamber space 16 is displaced to the right in that the first and the second plunger element 1, 2 are moved to the right, i.e. towards the further inlet 28, in a simultaneous manner (cf. Figure 5E). This movement is performed until the chamber space 16 is fluidly connected to the further inlet 28. Then, the chamber space 16 is also fluidly disconnected from the inlet 5. The first and the second plunger element 1, 2 are moved until the inner side face 18 of the second plunger element 1 is axially aligned with the right side face 23 of the further inlet 28 (cf. Figure 5E). The further inlet 28 is preferably fluidly connected to the substance reservoir 11 (cf. Figure 4). Then, the second plunger element 2 is locked and the first plunger element 1 is moved to the left and away from the second plunger element 2 (cf. Figure 5F). As the chamber space 16 is fluidly connected to the inlet 5, a further liquid 30 which may be retained in the substance reservoir, is moved into the chamber space 16 by an underpressure in the chamber 5 generated between the first and the second plunger element 1, 2. The second plunger element 2 is moved until a further subquantity 32 having a defined volume is moved into the chamber space 16 such that both, the liquid 12 and the further liquid 30 are placed or retained in the chamber space 16 (cf. Figure 5F). Consequently, the liquid 12 and the further liquid 30 will mix or blend and form a mixture 31.

In Figure 5G, the first and the second plunger element 1, 2 are moved simultaneously to the left, i.e. towards the inlet 5, until the inner side face 17 of the first plunger element 1 is aligned with a left side face 21 of the outlet 27 (cf. Figure 5H). Then, as shown in Figure 5H, the first plunger element 1 is locked and the second plunger element 2 is moved left, i.e. towards the first plunger element 1, in order to empty the chamber space 16. In Figure 5I, the inner side face 18 of the second plunger element 2 abuts the inner side face 17 of the first plunger element 1 and the chamber space 16 is completely emptied. Now, the medical pump 100 may be prepared to be operated again. In Figure 5J, the first and the second plunger element 1, 2 are simultaneously moved to the left, as compared to the Figure 5I.

The term "liquid" or "medical substance," as used herein, preferably means a pharmaceutical formulation containing at least one pharmaceutically active compound, wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
   H-(Lys)5-des Pro36, des Pro37 Exendin-4(1-39)-NH2,
   des Pro36 Exendin-4(1-39),
   des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(O2)25, Asp28] Exendin-4(1-39),
   des Pro36 [Trp(O2)25, IsoAsp28] Exendin-4(1-39),
   des Pro36 [Met(O)14 Trp(O2)25, Asp28] Exendin-4(1-39),
   des Pro36 [Met(O)14 Trp(O2)25, IsoAsp28] Exendin-4(1-39); or
des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(O2)25, Asp28] Exendin-4(1-39),
   des Pro36 [Trp(O2)25, IsoAsp28] Exendin-4(1-39),
   des Pro36 [Met(O)14 Trp(O2)25, Asp28] Exendin-4(1-39),
   des Pro36 [Met(O)14 Trp(O2)25, IsoAsp28] Exendin-4(1-39),
   wherein the group -Lys6-NH2 may be bound to the C-terminus of the Exendin-4 derivative;
or an Exendin-4 derivative of the sequence
   des Pro36 Exendin-4(1-39)-Lys6-NH2 (AVE0010),
   H-(Lys)6-des Pro36 [Asp28] Exendin-4(1-39)-Lys6-NH2,
   des Asp28 Pro36, Pro37, Pro38Exendin-4(1-39)-NH2,
   H-(Lys)6-des Pro36, Pro38 [Asp28] Exendin-4(1-39)-NH2,
   H-Asn-(Glu)5des Pro36, Pro37, Pro38 [Asp28] Exendin-4(1-39)-NH2,
   des Pro36, Pro37, Pro38 [Asp28] Exendin-4(1-39)-(Lys)6-NH2,
   H-(Lys)6-des Pro36, Pro37, Pro38 [Asp28] Exendin-4(1-39)-(Lys)6-NH2, H-Asn-(Glu)5-des Pro36, Pro37, Pro38 [Asp28] Exendin-4(1-39)-(Lys)6-NH2, H-(Lys)6-des Pro36 [Trp(O2)25, Asp28] Exendin-4(1-39)-Lys6-NH2,
   H-des Asp28 Pro36, Pro37, Pro38 [Trp(O2)25] Exendin-4(1-39)-NH2,
   H-(Lys)6-des Pro36, Pro37, Pro38 [Trp(O2)25, Asp28] Exendin-4(1-39)-NH2, H-Asn-(Glu)5-des Pro36, Pro37, Pro38 [Trp(O2)25, Asp28] Exendin-4(1-39)-NH2,
   des Pro36, Pro37, Pro38 [Trp(O2)25, Asp28] Exendin-4(1-39)-(Lys)6-NH2, H-(Lys)6-des Pro36, Pro37, Pro38 [Trp(O2)25, Asp28] Exendin-4(1-39)-(Lys)6-NH2, H-Asn-(Glu)5-des Pro36, Pro37, Pro38 [Trp(O2)25, Asp28] Exendin-4(1-39)-(Lys)6-NH2, H-(Lys)6-des Pro36 [Met(O)14, Asp28] Exendin-4(1-39)-Lys6-NH2,
   des Met(O)14 Asp28 Pro36, Pro37, Pro38 Exendin-4(1-39)-NH2,
   H-(Lys)6-desPro36, Pro37, Pro38 [Met(O)14, Asp28] Exendin-4(1-39)-NH2, H-Asn-(Glu)5-des Pro36, Pro37, Pro38 [Met(O)14, Asp28] Exendin-4(1-39)-NH2,
   des Pro36, Pro37, Pro38 [Met(O)14, Asp28] Exendin-4(1-39)-(Lys)6-NH2, H-(Lys)6-des Pro36, Pro37, Pro38 [Met(O)14, Asp28] Exendin-4(1-39)-(Lys)6-NH2, H-Asn-(Glu)5 des Pro36, Pro37, Pro38 [Met(O)14, Asp28] Exendin-4(1-39)-(Lys)6-NH2, H-Lys6-des Pro36 [Met(O)14, Trp(O2)25, Asp28] Exendin-4(1-39)-Lys6-NH2,
   H-des Asp28 Pro36, Pro37, Pro38 [Met(O)14, Trp(O2)25] Exendin-4(1-39)-NH2, H-(Lys)6-des Pro36, Pro37, Pro38 [Met(O)14, Asp28] Exendin-4(1-39)-NH2, H-Asn-(Glu)5-des Pro36, Pro37, Pro38 [Met(O)14, Trp(O2)25, Asp28] Exendin-4(1-39)-NH2,
   des Pro36, Pro37, Pro38 [Met(O)14, Trp(O2)25, Asp28] Exendin-4(1-39)-(Lys)6-NH2, H-(Lys)6-des Pro36, Pro37, Pro38 [Met(O)14, Trp(O2)25, Asp28] Exendin-4(S1-39)-(Lys)6-NH2,
   H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (-150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCI or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

The scope of protection of the invention is not limited to the examples given hereinabove. The invention is embodied in each novel characteristic and each combination of characteristics, which particularly includes every combination of any features which are stated in the claims, even if this feature or this combination of features is not explicitly stated in the claims or in the examples.

### Reference numerals

- 1: First plunger element
- 2: Second plunger element
- 3: Housing
- 4: Chamber
- 5: Inlet
- 6: Dispensing outlet
- 7: Non-dispensing outlet
- 8, 8': Stator
- 9, 9': Motor
- 10: Control Unit
- 11: Substance reservoir
- 12: Liquid
- 13: First side
- 14: Second side
- 15: Subquantity
- 16: Chamber space
- 17: Inner side face of the first plunger element
- 18: Inner side face of the second plunger element
- 19: Right side face of the inlet
- 20: Left side face of the dispensing outlet / inlet
- 21: Left side face of the inlet / outlet
- 22: Left side face of the non-dispensing outlet
- 23: Right side face of the non-dispensing outlet / further inlet
- 26: Content of the chamber space
- 27: Outlet
- 28: Further inlet
- 29: Sensor unit
- 30: Further liquid
- 31: Mixture
- 32: Further subquantity
- 100: Medical pump
- x: Longitudinal axis

## Claims

1. Medical pump (100) for dispensing a liquid (12), the medical pump (100) comprising:
- a chamber (4) further comprising an inlet (5) for receiving the liquid (12) and an outlet (27),
- a first plunger element (1) movably arranged in the chamber (4) and,
- a second plunger element (2) movably arranged in the chamber (4), the first plunger element (1) and the second plunger element (2) being movable with respect to one another to define a chamber space (16) formed between them, wherein the chamber space (16) is displaceable within the chamber (4) by a movement of the first and the second plunger element (1, 2), wherein the first and the second plunger element (1, 2) are arranged along a common longitudinal axis (x) and the chamber space (16) is axially displaceable by a simultaneous movement of the first and the second plunger element (1, 2) with respect to the chamber (4) and wherein the inlet (5) and the outlet (27) are arranged axially spaced apart from each other, wherein the medical pump (100) is operable and configured such that
- when the chamber space (16) is fluidly connected to the inlet, liquid (12) is moved into the chamber space (16) through the inlet (5) by a relative movement of the first and the second plunger element (1, 2) away from each other, and
- when liquid (12) is arranged in the chamber space (16) and the chamber space (16) is fluidly connected to the outlet (27), the liquid (12) is removed from the medical pump (100) through the outlet (27) by a relative movement of the first and the second plunger element (1, 2) towards each other,
**characterized in that**
the chamber (4) comprises an opening (33) which is arranged axially spaced from the inlet (5) and the outlet (27), wherein the chamber space (16) is fluidly connectable to and fluidly disconnectable from the opening (33) by movement of at least one of or both of the first and the second plunger element (1, 2) with respect to the chamber (4).

2. Medical pump (100) according to claim 1, wherein the chamber space (16) is fluidly connectable to and fluidly disconnectable from the inlet (5) and the outlet (27) by movement of at least one of or both of the first and the second plunger element (1, 2) with respect to the chamber (4).

3. Medical pump (100) according to claim 1 or 2, wherein at least one of or both of the first and the second plunger element (1, 2) is selectively lockable with respect to the chamber (4) such that, when the respective plunger element is locked, relative movement of that plunger element with respect to the chamber (4) is prevented.

4. Medical pump (100) according to at least one of the previous claims, comprising two motors (9, 9') which are operable to independently control the movement of the first and second plunger element (1, 2) within the chamber (4).

5. Medical pump (100) according to at least one of the previous claims, comprising a detection mechanism operable to detect whether a content (26) of the chamber space (16) which content comprises the liquid (12) fulfils a predetermined compression property requirement and, wherein the medical pump (100) is configured such that the detection mechanism is operated when the chamber space (16) is fluidly disconnected from the exterior of the chamber (4) and, wherein the outlet (27) is a dispensing outlet (6) and the opening (33) is a non-dispensing outlet (7).

6. Medical pump (100) according to claim 5, wherein, when the detection mechanism is operated, the content (26) of the chamber space (16) is compressed by relative movement of the first and the second plunger element (1, 2) with respect to each other, which relative movement tends to reduce or reduces the size of the chamber space (16), thereby compressing the content (26) of the chamber space (16).

7. Medical pump (100) according to claim 6, wherein the predetermined compression property requirement is fulfilled, when, during the operation of the detection mechanism, the content (26) of the chamber space (16) is compressed only up to a predetermined extent.

8. Medical pump (100) according to claim 7, wherein the predetermined extent is chosen such that, when the content (26) of the chamber space (16) is compressible more than the predetermined extent, a critical amount of gas is expected to be present in the chamber space (16) along with the liquid (12).

9. Medical pump (100) according to at least one of the claims 5 to 8, wherein the medical pump (100) is operable so as to dispense the liquid (12) through the outlet (6) if the predetermined compression property requirement is fulfilled, wherein the liquid (12) is dispensed by a relative movement of the first and the second plunger element (1, 2).

10. Medical pump (100) according to at least one of the claims 5 to 9, wherein the medical pump (100) is configured such that the detection mechanism is operated when the chamber space (16) is fluidly disconnected from the exterior of the chamber (4), and wherein the medical pump (100) is operable so as to empty the chamber space (16) through the opening (7) if the predetermined compression property requirement is not fulfilled, and wherein the content (26) of the chamber space (16) is removed by a relative movement of the first and the second plunger element (1, 2).

11. Medical pump (100) according to at least one of the claims 5 to 10, wherein the inlet (5) is arranged at an axial position between the outlet (27) and the opening (33), and wherein the detection mechanism is operated when the chamber space (16) is axially arranged between the inlet (5) and the outlet (27).

12. Medical pump (100) according to at least one of the previous claims, wherein the opening (33) is a further inlet (28) and the medical pump (100) is operable and configured such that a further liquid (30) is moved into the chamber space (16) through the opening (33) by a relative movement of the first and the second plunger element (1, 2) away from each other when the chamber space (16) is fluidly connected to the opening (33).

13. Medical pump (100) according to claim 12, wherein the medical pump (100) comprises a sensor unit (29) which is fluidly connected to the outlet (27), wherein the medical pump (100) is operable and configured such that when the liquid (12) and the further liquid (30) have been moved into the chamber space (16), the liquid (12) and the further liquid (30) mix and wherein the corresponding mixture (31) of the liquid (12) and the further liquid (30) is moved into the sensor unit (29).

## Patentansprüche

1. Medizinische Pumpe (100) zum Abgeben einer Flüssigkeit (12), wobei die medizinische Pumpe (100) Folgendes umfasst:
- eine Kammer (4), die ferner einen Einlass (5) zum Aufnehmen der Flüssigkeit (12) und einen Auslass (27) umfasst,
- ein erstes Kolbenelement (1), das beweglich in der Kammer (4) angeordnet ist, und
- ein zweites Kolbenelement (2), das beweglich in der Kammer (4) angeordnet ist, wobei das erste Kolbenelement (1) und das zweite Kolbenelement (2) in Bezug aufeinander beweglich sind, um einen Kammerraum (16) zu definieren, der zwischen diesen gebildet wird, wobei der Kammerraum (16) innerhalb der Kammer (4) durch eine Bewegung des ersten und des zweiten Kolbenelements (1, 2) verschieblich ist, wobei das erste und das zweite Kolbenelement (1, 2) entlang einer gemeinsamen Längsachse (x) angeordnet sind und der Kammerraum (16) durch eine gleichzeitige Bewegung des ersten und des zweiten Kolbenelements (1, 2) in Bezug auf die Kammer (4) axial verschieblich ist und wobei der Einlass (5) und der Auslass (27) axial voneinander beabstandet angeordnet sind, wobei die medizinische Pumpe (100) so betrieben werden kann und konfiguriert ist, dass
- wenn der Kammerraum (16) in Fluidverbindung mit dem Einlass steht, Flüssigkeit (12) in den Kammerraum (16) durch den Einlass (5) durch eine relative Bewegung des ersten und des zweiten Kolbenelements (1, 2) voneinander weg bewegt wird, und
- wenn sich Flüssigkeit (12) in dem Kammerraum (16) befindet und der Kammerraum (16) in Fluidverbindung mit dem Auslass (27) steht, die Flüssigkeit (12) aus der medizinischen Pumpe (100) durch den Auslass (27) durch eine relative Bewegung des ersten und des zweiten Kolbenelements (1, 2) aufeinander zu entfernt wird, **dadurch gekennzeichnet, dass** die Kammer (4) eine Öffnung (33) umfasst, die vom Einlass (5) und vom Auslass (27) axial beabstandet angeordnet ist, wobei der Kammerraum (16) mit der Öffnung (33) durch eine Bewegung mindestens eines aus dem ersten und dem zweiten Kolbenelement (1, 2) oder beider in Bezug auf die Kammer (4) fluidmäßig verbunden oder von dieser fluidmäßig getrennt werden kann.

2. Medizinische Pumpe (100) nach Anspruch 1, wobei der Kammerraum (16) mit dem Einlass (5) und dem Auslass (27) durch eine Bewegung mindestens eines aus dem ersten und dem zweiten Kolbenelement (1, 2) oder beider in Bezug auf die Kammer (4) fluidmäßig verbunden und von diesen fluidmäßig getrennt werden kann.

3. Medizinische Pumpe (100) nach Anspruch 1 oder 2, wobei mindestens eines aus dem ersten und dem zweiten Kolbenelement (1, 2) oder beide selektiv in Bezug auf die Kammer (4) verriegelt werden kann/können, so dass, wenn das entsprechende Kolbenelement verriegelt ist, eine relative Bewegung dieses Kolbenelements in Bezug auf die Kammer (4) verhindert wird.

4. Medizinische Pumpe (100) nach mindestens einem der vorstehenden Ansprüche, umfassend zwei Motoren (9, 9'), die betrieben werden können, um die Bewegung des ersten und des zweiten Kolbenelements (1, 2) innerhalb der Kammer (4) unabhängig zu steuern.

5. Medizinische Pumpe (100) nach mindestens einem der vorstehenden Ansprüche, umfassend einen Erkennungsmechanismus, der betätigt werden kann, um zu erkennen, ob ein Inhalt (26) des Kammerraums (16), wobei der Inhalt die Flüssigkeit (12) umfasst, eine vorbestimmte Kompressionseigenschaftsanforderung erfüllt, und wobei die medizinische Pumpe (100) so konfiguriert ist, dass der Erkennungsmechanismus betätigt wird, wenn der Kammerraum (16) fluidmäßig von dem Äußeren der Kammer (4) getrennt ist, und wobei der Auslass (27) ein Abgabeauslass (6) ist und die Öffnung (33) ein Nicht-Abgabeauslass (7) ist.

6. Medizinische Pumpe (100) nach Anspruch 5, wobei, wenn der Erkennungsmechanismus betätigt wird, der Inhalt (26) des Kammerraums (16) durch eine relative Bewegung des ersten und des zweiten Kolbenelements (1, 2) in Bezug aufeinander komprimiert wird, wobei die relative Bewegung darauf abzielt, die Größe des Kammerraums (16) zu verringern, oder diese verringert, wodurch der Inhalt (26) des Kammerraums (16) komprimiert wird.

7. Medizinische Pumpe (100) nach Anspruch 6, wobei die vorbestimmte Kompressionseigenschaftsanforderung erfüllt ist, wenn während der Betätigung des Erkennungsmechanismus der Inhalt (26) des Kammerraums (16) nur bis zu einem vorbestimmten Grad komprimiert ist.

8. Medizinische Pumpe (100) nach Anspruch 7, wobei der vorbestimmte Grad so ausgewählt ist, dass, wenn der Inhalt (26) des Kammerraums (16) mehr als zu dem vorbestimmten Grad komprimierbar ist, voraussichtlich eine kritische Menge Gas im Kammerraum (16) zusammen mit der Flüssigkeit (12) vorhanden ist.

9. Medizinische Pumpe (100) nach mindestens einem der Ansprüche 5 bis 8, wobei die medizinische Pumpe (100) so betätigt werden kann, dass sie die Flüssigkeit (12) durch den Auslass (6) abgibt, wenn die vorbestimmte Kompressionseigenschaftsanforderung erfüllt ist, wobei die Flüssigkeit (12) durch eine relative Bewegung des ersten und des zweiten Kolbenelements (1, 2) abgegeben wird.

10. Medizinische Pumpe (100) nach mindestens einem der Ansprüche 5 bis 9, wobei die medizinische Pumpe (100) so konfiguriert ist, dass der Erkennungsmechanismus betätigt wird, wenn der Kammerraum (16) fluidmäßig vom Äußeren der Kammer (4) getrennt ist, und wobei die medizinische Pumpe (100) so betätigt werden kann, dass sie den Kammerraum (16) durch die Öffnung (7) entleert, wenn die vorbestimmte Kompressionseigenschaftsanforderung nicht erfüllt ist, und wobei der Inhalt (26) des Kammerraums (16) durch eine relative Bewegung des ersten und des zweiten Kolbenelements (1, 2) entfernt wird.

11. Medizinische Pumpe (100) nach mindestens einem der Ansprüche 5 bis 10, wobei der Einlass (5) an einer axialen Position zwischen dem Auslass (27) und der Öffnung (33) angeordnet ist und wobei der Erkennungsmechanismus betätigt wird, wenn der Kammerraum (16) axial zwischen dem Einlass (5) und dem Auslass (27) angeordnet ist.

12. Medizinische Pumpe (100) nach mindestens einem der vorstehenden Ansprüche, wobei die Öffnung (33) ein weiterer Einlass (28) ist und die medizinische Pumpe (100) so betätigt werden kann und konfiguriert ist, dass eine weitere Flüssigkeit (30) in den Kammerraum (16) durch die Öffnung (33) durch eine relative Bewegung des ersten und des zweiten Kolbenelements (1, 2) voneinander weg bewegt wird, wenn der Kammerraum (16) fluidmäßig mit der Öffnung (33) verbunden ist.

13. Medizinische Pumpe (100) nach Anspruch 12, wobei die medizinische Pumpe (100) eine Sensoreinheit (29) umfasst, die in Fluidverbindung mit dem Auslass (27) steht, wobei die medizinische Pumpe (100) so betätigt werden kann und konfiguriert ist, dass, wenn die Flüssigkeit (12) und die weitere Flüssigkeit (30) in den Kammerraum (16) bewegt worden sind, die Flüssigkeit (12) und die weitere Flüssigkeit (30) sich vermischen, und wobei die entsprechende Mischung (31) der Flüssigkeit (12) und der weiteren Flüssigkeit (30) in die Sensoreinheit (29) bewegt wird.

## Revendications

1. Pompe médicale (100) destinée à administrer un liquide (12), la pompe médicale (100) comprenant :
- une chambre (4) comprenant en outre une admission (5) destinée à recevoir le liquide (12) et une évacuation (27),
- un premier élément de piston (1) agencé de manière mobile dans la chambre (4) et,
- un deuxième élément de piston (2) agencé de manière mobile dans la chambre (4), le premier élément de piston (1) et le deuxième élément de piston (2) étant mobiles l'un par rapport à l'autre pour définir un espace de chambre (16) formé entre eux, dans laquelle l'espace de chambre (16) est déplaçable à l'intérieur de la chambre (4) par un mouvement des premier et deuxième éléments de piston (1, 2), dans laquelle les premier et deuxième éléments de piston (1, 2) sont agencés le long d'un axe longitudinal commun (x) et l'espace de chambre (16) est déplaçable axialement par un mouvement simultané des premier et deuxième éléments de piston (1, 2) par rapport à la chambre (4) et dans laquelle l'admission (5) et l'évacuation (27) sont agencées axialement espacées l'une de l'autre, dans laquelle la pompe médicale (100) est actionnable et configurée de manière à ce que
- quand l'espace de chambre (16) est connecté par voie fluide à l'admission, le liquide (12) est amené dans l'espace de chambre (16) à travers l'admission (5) par un mouvement relatif des premier et deuxième éléments de piston (1, 2) en éloignement l'un de l'autre, et
- quand le liquide (12) est agencé dans l'espace de chambre (16) et que l'espace de chambre (16) est connecté par voie fluide à l'évacuation (27), le liquide (12) est retiré de la pompe médicale (100) à travers l'évacuation (27) par un mouvement relatif des premier et deuxième éléments de piston (1, 2) l'un vers l'autre,
**caractérisé en ce que** la chambre (4) comprend une ouverture (33) qui est agencée espacée axialement de l'admission (5) et de l'évacuation (27), dans laquelle l'espace de chambre (16) est connectable par voie fluide à et déconnectable par voie fluide de l'ouverture (33) par le mouvement d'au moins l'un ou des deux des premier et deuxième éléments de piston (1, 2) par rapport à la chambre (4).

2. Pompe médicale (100) selon la revendication 1, dans laquelle l'espace de chambre (16) est connectable par voie fluide à et déconnectable par voie fluide de l'admission (5) et de l'évacuation (27) par un mouvement d'au moins l'un ou des deux des premier et deuxième éléments de piston (1, 2) par rapport à la chambre (4).

3. Pompe médicale (100) selon la revendication 1 ou 2, dans laquelle au moins l'un ou les deux des premier et deuxième éléments de piston (1, 2) est verrouillable sélectivement par rapport à la chambre (4) de manière à ce que, quand l'élément de piston respectif est verrouillé, un mouvement relatif de cet élément de piston par rapport à la chambre (4) soit empêché.

4. Pompe médicale (100) selon au moins l'une des revendications précédentes, comprenant deux moteurs (9, 9') qui sont actionnables pour commander indépendamment le mouvement des premier et deuxième éléments de piston (1, 2) à l'intérieur de la chambre (4).

5. Pompe médicale (100) selon au moins l'une des revendications précédentes, comprenant un mécanisme de détection actionnable pour détecter si un contenu (26) de l'espace de chambre (16) lequel contenu comprend le liquide (12) remplit une exigence de propriété de compression prédéterminée et, dans laquelle la pompe médicale (100) est configurée de manière à ce que le mécanisme de détection soit actionné quand l'espace de chambre (16) est déconnecté par voie fluide depuis l'extérieur de la chambre (4) et, dans lequel l'évacuation (27) est une évacuation d'administration (6) et l'ouverture (33) est une évacuation non d'administration (7).

6. Pompe médicale (100) selon la revendication 5, dans laquelle, quand le mécanisme de détection est actionné, le contenu (26) de l'espace de chambre (16) est compressé par un mouvement relatif des premier et deuxième éléments de piston (1, 2) l'un par rapport à l'autre, lequel mouvement relatif tend à réduire ou réduit la taille de l'espace de chambre (16), comprimant de la sorte le contenu (26) de l'espace de chambre (16).

7. Pompe médicale (100) selon la revendication 6, dans laquelle l'exigence de propriété de compression prédéterminée est remplie, quand, en cours d'actionnement du mécanisme de détection, le contenu (26) de l'espace de chambre (16) est compressé uniquement jusqu'à une mesure prédéterminée.

8. Pompe médicale (100) selon la revendication 7, dans laquelle la mesure prédéterminée est choisie de manière à ce que, quand le contenu (26) de l'espace de chambre (16) est compressible plus que la mesure prédéterminée, la présence d'une quantité critique de gaz soit prévue dans l'espace de chambre (16) conjointement au liquide (12).

9. Pompe médicale (100) selon au moins l'une des revendications 5 à 8, dans laquelle la pompe médicale (100) est actionnable de façon à administrer le liquide (12) à travers l'évacuation (6) si l'exigence de propriété de compression prédéterminée est remplie, dans laquelle le liquide (12) est administré par un mouvement relatif des premier et deuxième éléments de piston (1, 2).

10. Pompe médicale (100) selon au moins l'une des revendications 5 à 9, dans laquelle la pompe médicale (100) est configurée de manière à ce que le mécanisme de détection soit actionné quand l'espace de chambre (16) est déconnecté par voie fluide depuis l'extérieur de la chambre (4), et dans laquelle la pompe médicale (100) est actionnable de façon à vider l'espace de chambre (16) à travers l'ouverture (7) si l'exigence de propriété de compression prédéterminée n'est pas remplie, et dans laquelle le contenu (26) de l'espace de chambre (16) est retiré par un mouvement relatif des premier et deuxième éléments de piston (1, 2).

11. Pompe médicale (100) selon au moins l'une des revendications 5 à 10, dans laquelle l'admission (5) est agencée à une position axiale entre l'évacuation (27) et l'ouverture (33), et dans laquelle le mécanisme de détection est actionné quand l'espace de chambre (16) est agencé axialement entre l'admission (5) et l'évacuation (27).

12. Pompe médicale (100) selon au moins l'une des revendications précédentes, dans laquelle l'ouverture (33) est une admission plus éloignée (28) et la pompe médicale (100) est actionnable et configurée de manière à ce qu'un liquide supplémentaire (30) soit amené dans l'espace de chambre (16) à travers l'ouverture (33) par un mouvement relatif des premier et deuxième éléments de piston (1, 2) en éloignement l'un de l'autre quand l'espace de chambre (16) est connecté par voie fluide à l'ouverture (33).

13. Pompe médicale (100) selon la revendication 12, dans laquelle la pompe médicale (100) comprend une unité de capteur (29) qui est connectée par voie fluide à l'évacuation (27), dans laquelle la pompe médicale (100) est actionnable et configurée de manière à ce que quand le liquide (12) et le liquide supplémentaire (30) n'ont pas été amenés dans l'espace de chambre (16), le liquide (12) et le liquide supplémentaire (30) se mélangent et dans laquelle le mélange correspondant (31) du liquide (12) et du liquide supplémentaire (30) est amené dans l'unité de capteur (29).
